# EUROPEAN PATENT APPLICATION

(11) **EP 4 163 367 A1**
(43) Date of publication of application: **12.04.2023**
(21) Application number: 21821487.2
(22) Date of filing: 07.06.2021
(51) Int. Cl.: C12N 5/0783, C07K 14/54

(54) **GENETICALLY MANIPULATED CELL STRAIN FOR ACTIVATING AND AMPLIFYING NK CELLS AND USE THEREOF**

(30) Priority: 09.06.2020 KR 20200069854; 08.04.2021 KR 20210046106
(71) Applicant: Samsung Life Public Welfare Foundation, Seoul 04348 (KR); Seoul National University R & DB Foundation, Seoul 08826 (KR)
(72) Inventor: CHO, Duck, Seoul 06603 (KR); DOH, Junsang, Seoul 06597 (KR); PHAN, Thi Minh-Trang, Seoul 05667 (KR); KIM, Jinho, Seoul 06342 (KR)
(74) Representative: Patentanwälte Olbricht Buchhold Keulertz
(86) International application number: PCT/KR2021/007092
(87) International publication number: WO 2021/251708

(57) **Abstract**

Provided is a cell genetically engineered for activating natural killer (NK) cells. The genetically engineered cell for activating NK cells according to an aspect may synergistically induce the proliferation and activation of NK cells in a sample, thereby producing effects that can be usefully utilized in a method of proliferating NK cells, a method of measuring NK cells proliferated by the method or an activation degree of NK cells, or a method of diagnosing an NK cell activity-related disease.

## Description

### TECHNICAL FIELD

The present application is based on and claims priority to Korean Patent Application Nos. 10-2020-0069854, filed on June 9, 2020, and 10-2021-0046106, filed on April 8, 2021, in the Korean Intellectual Property Office, the disclosures of which are incorporated by reference herein in their entirety.

The present application relates to a cell line genetically engineered to activate and expand NK cells and use of the cell line, and more particularly, to a method proliferating NK cells by using the cell line or a method of measuring activity of NK cells by using the cell line.

### BACKGROUND ART

Natural killer (NK) cells are a type of cytotoxic lymphocytes important for innate immunity. NK cells respond to virus-infected cells or cancer cells, and such responses are determined by signals generated by a reaction between a number of activating receptors and inhibitory receptors of the NK cells and respective ligands of a target cell. Typically, when an activating signal is more dominant than an inhibitory signal, the NK cells can attack a target cell, whereas when an inhibitory signal is more dominant than an activating signal, the NK cells do not attack a target cell. Therefore, since normal healthy cells having an inhibitory receptor ligand (e.g., major histocompatibility complex (MHC)) for the NK cells produce inhibitory signals, the normal healthy cells are not attacked by the NK cells.

Some cancer cells may have MHC abnormalities on the cellular surface, and thus the number of the cancer cells may decrease. In this case, no inhibitory signal for the NK cells is produced, and thus the NK cells attack a target cell. The NK cells secrete perforin to form a pore in a cell membrane of an infected cell or a cancer cell, and then releases a granzyme to kill the cell, resulting in cytotoxicity. In the case of patients having B-cell lymphoma and many other cancer patients, defects in the number or anticancer activity of the NK cells have been found, and such dysfunction of NK cells is known to be closely related to the occurrence of cancer.

Therefore, as use of the anticancer ability of the NK cells is emerging as a treatment for cancer patients, the development of an expansion method to secure a large number of high-performance NK cells is important. In addition, people with NK cell dysfunction or abnormalities are known to be associated with cancer and various diseases, and thus there is a demand for the development of a method of measuring activity of the NK cells.

The previously reported methods of proliferating NK cells use various expensive cytokines at high concentrations or use peripheral blood mononuclear cells or cancer cell lines in combination. However, these methods not only require high costs, but also result in expansion rates that are not very high. In addition, in many studies, besides NK cells, there is also the expansion of other lymphocytes such as T cells, showing unsuitability for NK cell-based immune cell therapy. Therefore, the development of an efficient method of selectively expanding only NK cells is a major challenge and is being researched (Korean Patent No. 10-1525199), but the situation is still inadequate.

Meanwhile, the cytotoxicity examination among NK cell activation examinations is a method of examining a function of NK cells to directly kill a targeted cancer cell (e.g., K562 cell line), and is currently used in clinical laboratories. Recently, a cytotoxicity examination using whole blood that does not require a cell isolation process, instead of using peripheral blood mononuclear cells that require a complicated cell isolation process, has been introduced. However, considering that the use of whole blood requires various types of cytokines, the problem of increasing examination costs has emerged and needs to be overcome.

### DESCRIPTION OF EMBODIMENTS

### TECHNICAL PROBLEM

An aspect provides a cell line or feeder cell genetically engineered to express membrane bound interleukin-18 (mbIL-18) and membrane bound interleukin-21 (mblL-21).

Another aspect provides a method of proliferating a natural killer (NK) cell, the method including: obtaining a blood sample including a population of mixed NK cells (e.g., mixed immune cells); and contacting at least a portion of the population of NK cells with a cell genetically engineered to activate NK cells, wherein the genetically engineered cell is genetically engineered to express membrane bound interleukin-18 (mblL-18) and membrane bound interleukin-21 (mbIL-21).

Another aspect provides a method of examining an activation degree of NK cells, the method including: obtaining a blood sample including a population of mixed NK cells (e.g., mixed immune cells); contacting at least a portion of the population of NK cells with a cell genetically engineered to activate NK cells to activate the NK cells, the genetically engineered cell being genetically engineered to express mbIL-18 and mblL-21; and analyzing an activation degree of the activated NK cells.

Another aspect provides a method of diagnosing an NK cell-related disease or providing information on diagnosis of an NK cell-related disease, the method including: obtaining a blood sample including a population of mixed NK cells (e.g., mixed immune cells); contacting at least a portion of the population of NK cells with a cell genetically engineered to activate NK cells to activate the NK cells, the genetically engineered cell being genetically engineered to express mbIL-18 and mblL-21; and analyzing an activation degree of the activated NK cells.

### SOLUTION TO PROBLEM

An aspect provides a cell line genetically engineered to express membrane bound interleukin-18 (mbIL-18) and membrane bound interleukin-21 (mbIL-21).

The cell line may be used as a feeder cell that selectively expands only NK cells. In general, a K562 cell line, which is a representative nutritional feeder cell used for proliferation of NK cells, is only used for proliferation of NK cells, and thus the K562 cell line itself should not proliferate. Therefore, before culturing with the NK cells, the K562 cell line may be pre-treated through strong irradiation (e.g., in a range of about 50 gray (Gy) to about 100 Gy) so that the K562 cell line itself does not proliferate at all, but helps proliferation of NK cells only. Meanwhile, for the efficient proliferation of the NK cells, most cytokines, such as IL-2, IL-15, and the like, must be continuously exposed to the NK cells. Therefore, when a cell is genetically engineered to express cytokines, such as IL-2 and/or IL-15 in a cancer cell strain-based nutritional feeder cell, a cancer cell line that is pre-treated with irradiation or the like itself cannot survive for a long period of time during a culture process, thereby degrading selective proliferation efficiency of the NK cells. In an embodiment, it is confirmed that, when the NK cells are exposed to IL-18 or IL-21 for a short period of time in the presence of the K562 cell line, the cell expansion effect does not appear until Day 21 of the culture, but when the NK cells are exposed to IL-18 and IL-21 simultaneously, the cell expansion effect appears after Day 28 of the culture. Accordingly, it is confirmed that, in the case of IL-18 and IL-21, only single and short exposure on the starting day of the culture is enough to exhibit an effect in the proliferation of the NK cells despite only one exposure and short-term exposure on the starting day of culture. That is, since the feeder cell line genetically engineered to express mbIL-18 and mbIL-21 according to an aspect does not reduce the proliferation efficiency of the NK cells despite of short-term expression of IL-18 and IL-21, only the NK cells can be selectively expanded.

The term "feeder cell (also referred to as a supporting cell)" as used in the present specification refers to a cell that does not have the ability to divide due to irradiation, but has metabolic activity so that it produces various metabolites and helps target NK cells proliferated. For the feeder cell available in the present specification, an animal cell line into which a gene is introduced, such as a human chronic myelogenous leukemia cell line (e.g., K562 cell), RPMI8866, EBV_LCL, 721.221, HFWT, NK-92, and the like may be used.

The term "genetic engineering" or "genetically engineered" as used in the present specification refers to the act of introducing one or more genetic modifications into a cell, or to a cell produced by the act.

Particularly, the genetically engineered cell may include an exogenous gene encoding a referenced gene. The term "exogenous" refers that a referenced molecule or referenced activity has been introduced into a host cell. For example, the molecule may be introduced into a host genetic material of an encoding nucleic acid, such as insertion into a host chromosome, or may be introduced as a non-chromosomal genetic material including a plasmid. Regarding expression of the encoding nucleic acid, the term "exogenous" refers that the encoding nucleic acid is introduced into a subject in an expressible form. Regarding biosynthetic activity, the term "exogenous" refers to the activity introduced into a host parent cell. A source of the activity may be, for example, a homologous or heterologous encoding nucleic acid that expresses the referenced activity after introduction into the host parent cell. In this regard, the term "endogenous" refers to the referenced molecule or referenced activity present in the host cell. Similarly, regarding expression of the encoding nucleic acid, the term "endogenous" refers to expression of the encoding nucleic acid included in a subject. The term "heterologous" refers to a molecule or activity from a source other than the referenced species, and the term "homologous" refers to a molecule or activity from the host parent cell. Accordingly, the exogenous expression of the encoding nucleic acid may utilize either or both heterologous and homologous encoding nucleic acids.

Therefore, the cell may include a nucleic acid encoding mbIL-18 and mblL-21. More particularly, the cell may be transformed with a vector including a nucleic acid encoding mbIL-18 and mblL-21.

The term "vector" as used in the present specification is a vector capable of expressing a target protein in a suitable host cell, and refers to a genetic construct including regulatory factors operably linked to express a gene insert. A vector according to an embodiment may include expression regulatory factors, such as a promoter, an operator, a start codon, a stop codon, a polyadenylation signal, and/or an enhancer, and the promoter of the vector may be constitutive or inducible. In addition, the vector may be an expression vector capable of stably expressing the fusion protein in a host cell. For the expression vector, a conventional vector in the art used to express a foreign protein in plants, animals, or microorganisms may be used. A recombinant vector may be constructed through various methods known in the art. For example, the vector may include a selectable marker for selecting a host cell including a vector, and in the case of a replicable vector, it may include an origin of replication. In addition, the vector may be self-replicated or introduced into host DNA, wherein the vector may be selected from the group consisting of a plasmid, a lentivirus, an adenovirus, an adeno-associated virus, a retrovirus, a herpes simplex virus, and a vaccinia viruses.

In addition, in the vector, a polynucleotide sequence encoding the above-described fusion protein may be operably linked to the promoter. The term "operably linked" as used in the present specification refers to a functional linkage between a regulatory sequence for the nucleic acid expression control sequence (e.g., a promoter, a signal sequence, or an array of transcriptional regulator binding sites) and another nucleic acid sequence. In this regard, the regulatory sequence may regulate transcription and/or translation of the another nucleic acid sequence.

The term "membrane bound interleukin" as used in the present specification refers to an interleukin bound to a cell membrane, and may have a meaning distinct from secretion of interleukin to the outside of the cell. mbIL-18 may have sequence homology of greater than or equal to about 70 %, greater than or equal to about 75 %, greater than or equal to about 80 %, greater than or equal to about 85 %, greater than or equal to about 90 %, greater than or equal to about 92 %, greater than or equal to about 95 %, greater than or equal to about 97 %, greater than or equal to about 98 %, or greater than or equal to about 99 %, with respect to the nucleic acid sequence of SEQ ID NO: 1 or the amino acid sequence thereof. mbIL-21 may have sequence homology of greater than or equal to about 70 %, greater than or equal to about 75 %, greater than or equal to about 80 %, greater than or equal to about 85 %, greater than or equal to about 90 %, greater than or equal to about 92 %, greater than or equal to about 95 %, greater than or equal to about 97 %, greater than or equal to about 98 %, or greater than or equal to about 99 %, with respect to the nucleic acid sequence of SEQ ID NO: 2 or the amino acid sequence thereof.

Another aspect provides a composition for culturing NK cells, the composition including a cell genetically engineered to express mbIL-18 and mblL-21.

Specific details of the genetically engineered cell are as described above.

The composition including cells genetically engineered to express mbIL-18 and mbIL-21 for culturing NK cells according to an embodiment may induce expansion and/or activation of NK cells (e.g., natural killer cells), and thus the composition may be usefully used for culturing, isolation, or proliferation of NK cells.

Another aspect provides a method of proliferating NK cells, the method including: obtaining a blood sample including a population of mixed NK cells (e.g., mixed immune cells); and contacting at least a portion of the population of mixed NK cells with a cell genetically engineered to activate NK cells, wherein the genetically engineered cell is genetically engineered to express mbIL-18 and mblL-21.

In an embodiment, the contacting may include co-culturing the genetically engineered cell and the population of mixed NK cells to stimulate, activate, or expand a subpopulation of the NK cells.

The term "stimulation of NK cells" as used in the present specification refers that the activity, for example, the cytotoxic activity, of the NK cells *in vitro* or *in vivo* is increased or that the activated NK cells are generated, increased, expanded, or proliferated.

In an embodiment, non-limiting examples of the NK cells include macrophages, B lymphocytes, T lymphocytes, mastocytes, monocytes, dendritic cells, eosinophils, natural killer cells, basophils, neutrophils, and the like. Thus, in particular embodiments, the NK cells may be one selected from the group consisting of macrophages, B lymphocytes, T lymphocytes (CD8+ CTL), mastocytes, monocytes, dendritic cells, eosinophils, natural killer cells, basophils, and neutrophils. In particular embodiments, the NK cells may be natural killer cells or T lymphocytes. The mixed NK cells may include one or more selected from the group consisting of macrophages, B lymphocytes, T lymphocytes, mastocytes, monocytes, dendritic cells, eosinophils, natural killer cells, basophils, and neutrophils.

The term "natural killer cells" or "NK cells" as used herein refers to cytotoxic lymphocytes constituting major components of the innate immune system, and are defined as large granular lymphocytes (LGLs) that constitute other cells differentiated from B and T lymphocytes that are produced from common lymphoid progenitors (CLPs). The "natural killer cells" or "NK cells" include natural killer cells having no further modification derived from any tissue source, and may include mature natural killer cells as well as natural killer progenitor cells. The natural killer cells are activated in response to interferons or macrophage-derived cytokines, and include two types of surface receptors labeled as "an activating receptor" and "an inhibitory receptor", which control the cytotoxic activity of cells. The natural killer cells may be produced from hematoblasts, such as hemopoietic stem cells or precursors, of any source, such as placenta tissue, placenta perfusion, cord blood, placental blood, peripheral blood, and spleen, liver, and the like.

In an embodiment, the natural killer cells may be activated natural killer cells. The activated natural killer cells may refer to cells in which the cytotoxicity or the intrinsic immunomodulatory ability of natural killer cells is activated, compared to mother cells, such as hematopoietic cells, or progenitors of natural killer cells.

In an embodiment, the activated natural killer cells or an enriched population of the activated natural killer cells may be evaluated by detecting at least one functionally related marker, such as CD16, CD57, CD69, CD94, CD161, CD158a, CD158b, NKp30, NKp44, NKp46, DNAM-1, 2B4, NKp46, CD94, KIR (e.g., KIR2DL1, KIR2DL2/3, KIR3DL1, etc.), and NKG2 family (e.g., NKG2A, NKG2C, NKG2D, etc.) of the activating receptor.

In an embodiment, the co-culture may be performed in the presence of cytokines.

The term "cytokine" as used herein refers to a protein (5 kDa to 20 kDa) that has a role in cell signaling. Cytokines are released by cells and affect the behavior of cytokine-releasing cells and/or other cells. Non-limiting examples of cytokines include chemokines, interferons, interleukins, lymphokines, tumor necrosis factors, monokines, and colony-stimulating factors. Cytokines may be produced by a wide range of cells including (but not limited to) immune cells, such as macrophages, B lymphocytes, T lymphocytes, mastocytes, and monocytes, endothelial cells, fibroblasts, and interstitial cells. Cytokines may be produced by more than one type of cells. Cytokines act through receptors and are of particular importance in the immune system, and also regulate the balance between humoral and cellular immune responses and regulate the maturation, growth and responsiveness of cell populations. Cytokines used in the present specification may be naturally occurring cytokines or mutated version of naturally occurring cytokines. The term ""naturally occurring" as used herein may also be referred to as a wild-type and include allelic variants. The mutated version or "mutation" of a naturally occurring cytokine refers to specific mutations on a naturally occurring sequence to alter the function, activity, and/or specificity of the cytokines. In an embodiment, the mutations may enhance the function, activity, and/or specificity of the cytokines. In one or more embodiments, the mutations may decrease the function, activity, and/or specificity of the cytokines. The mutations may include deletions or additions of one or more amino acid residues of the cytokines.

The cytokine may include one selected from the group consisting of bone morphogenetic protein (BMP) family, cheomkine ligand (CCL) family, CKLF-like MARVEL transmembrane domain containing member (CMTM) family, C-X-C motif ligand (CXCL) family, growth/differentiation factor (GDF) family, growth hormone, interferon (IFN) family, interleukin (IL) family, tumor necrosis factor (TNF) family, glycophosphatidylinositol (GPI), secreted *Ly-6*/*uPAR-*related protein 1 (SLUPR-1), secreted *Ly-6*/*uPAR-*related protein 2 (SLUPR-2), and a combination thereof.

In an embodiment, the cytokine may be an interleukin or a mutation thereof. Many interleukins are synthesized by not only CD4 helper T lymphocytes, but also monocytes, macrophages, and endothelial cells. Interleukins may promote the development and differentiation of T and B lymphocytes and hematoblasts. Non-limiting examples of the interleukins (IL) include IL1, IL2, IL3, IL4, IL5, IL6, IL7, IL8 (CXCL8), IL9, IL10, IL11, IL12, IL13, IL14, IL15, IL16, IL17, IL18, IL19, IL20, IL21, IL22, IL23, IL24, IL25, IL26, IL27, IL28, IL29, IL30, IL31, IL32, IL33, IL35, IL36, and the like. Thus, in one or more embodiments, the cytokine may be a wild-type or mutant-type (but not limited thereto) of IL1, IL2, IL3, IL4, IL5, IL6, IL7, IL8 (CXCL8), IL9, IL10, IL11, IL12, IL13, IL14, IL15, IL16, IL17, IL18 , IL19, IL20, IL21, IL22, IL23, IL24, IL25, IL26, IL27, IL28, IL29, IL30, IL31, IL32, IL33, IL35, or IL36, or a mutation of these examples.

In one or more embodiments, the cytokines added for the co-culture may be IL-18 and IL-21. More specifically, the co-culture may include adding IL-18 and IL-21 to a culture medium while culturing in the presence of IL-2 and IL-15. The IL-21 may be used at a concentration in a range of about 1 ng/ml to about 20 ng/ml, about 1 ng/ml to about 15 ng/ml, about 1 ng/ml to about 10 ng/ml, or about 2 ng/ml to about 8 ng/ml. The IL-18 may be used at a concentration in a range of about 10 ng/ml to about 200 ng/ml, about 10 ng/ml to about 180 ng/ml, about 20 ng/ml to about 160 ng/ml, about 20 ng/ml to about 120 ng/ml, about 20 ng/ml to about 40 ng/ml, about 20 ng/ml to about 80 ng/ml, about 40 ng/ml to about 160 ng/ml, about 40 ng/ml to about 120 ng/ml, about 40 ng/ml to about 80 ng/ml, about 80 ng/ml to about 160 ng/ml, or about 80 ng/ml to about 120 ng/ml. In an embodiment, by exposing the cells to IL-18 and IL-21 during the co-culture, the proliferation of NK cells may be further synergistically increased.

In an embodiment, the co-culture may be performed for 2 days to 30 days.

In an embodiment, the genetically engineered cells may be treated with radiation in a range of about 50 gray (Gy) to about 300 Gy.

In an embodiment, the sample may be a biological sample derived from a subject, for example, a mammal including a human. In addition, the biological sample may be isolated from a subject, and may be blood, whole blood, serum, plasma, lymph, urine, feces, tissue, cell, organ, bone marrow, saliva, sputum, cerebrospinal fluid, or a combination thereof. In addition, the biological sample may include PBMCs, purified NK cells, or primary resting cells (i.e., cells directly isolated from blood).

Another aspect provides a method of testing an activation degree of NK cells, the method including: obtaining a blood sample including a population of mixed NK cells; contacting at least a portion of the population of mixed NK cells with cells that are genetically engineered to activate the NK cells to activate the NK cells, wherein the cells that are genetically engineered are genetically engineered to express mbIL-18 and mblL-21; and analyzing an activation degree of the activated NK cells.

Another aspect provides a method of diagnosing an NK cell-related disease or providing information on diagnosis of an NK cell-related disease, the method including: obtaining a blood sample including a population of mixed NK cells; contacting at least a portion of the population of NK cells with cells genetically engineered to activate the NK cells to activate the NK cells, wherein the cells that are genetically engineered are genetically engineered to express mbIL-18 and mblL-21; and analyzing an activation degree of the activated NK cells.

In the method of examining the activation degree of NK cells of the present invention, the step of comparing the activation degree of the NK cells with normal NK cells includes, specifically when the normal NK cells as a control are stimulated by the above-described genetically engineered cells under the same conditions as an experimental group, determining abnormal in cases where an activation phenomenon shown in the normal NK cells is significantly high, does not appear, or is significantly low in the experimental group. By the steps above, abnormal pathological signs of NK cell-related diseases, viral infection, the presence of cancer cells, and specific cancers may be determined, and accordingly, may predict the prognosis for these diseases. The "normal NK cells" refers to NK cells belonging to or derived from a subject without a disease, wherein the subject without a disease does not have at least a physical, genetic, or exogenous disease known to affect the activity of NK cells.

In an embodiment, the method may further include isolating the required NK cells from the sample. When necessary, the further included step may be performed after stimulation in the presence of other blood cells or lymphocytes, and the stimulation may be performed after the further included step is performed to obtain a sample containing only NK cells as lymphocytes. The degree of purification of the isolated NK cells and the composition of the sample may be varied to the extent required for the experiment. The NK cells may be used as purified from the sample as necessary, and may be used after being proliferated to secure conditions or cell quantities suitable for the experiment.

However, considering that a combination of specific factors is specific to the NK cell, when a combination of factors specific to the NK cells in the method of the present invention, the step of isolating the NK cells may not be essential.

In an embodiment, the step of measuring the degree of activation may include measuring one or more selected from degranulation activity, cytotoxic activity, cytokines secreted by the stimulation of NK cells.

The degranulation activity may refer to lysis of a target cell induced by secretion of, for example, perforin or granzyme, and may be analyzed by using FACS. In detail, after stimulating PBMCs isolated from a whole blood sample or purely isolated NK cells, a method of measuring CD107a expression proportional to degranulation by using a fluorochrome-conjugated antibody may be used.

The cytotoxic activity may be, for example, measured by measuring the amount of fluorescent dye released through lysis of target cells with a microplate reader after incubation with a culture including target cells capable of activating NK cells labeled with europium fluorescent dye.

In an embodiment, the cytokine may be selected from IFN-γ, TNF-α, TNF-β, MIP-1α, MIP-1βPANTES, IL-8, and IL-10. The analysis of the expression of the immunoactivator of NK cells as described above may be performed by using FACS, intracellular cytokine staining, ELISA, or the like. In detail, regarding the analysis, a fluorochrome-conjugated antibody may be used to stain the surface of the NK cells, and then the resulting cells are subjected to permeabilization while a different specific fluorochrome-conjugated immunoactivating factor (e.g., IFN-γ antibody) may be used to stain cytokines or the like, so as to measure the expression of immune activation factors in NK cells.

In an embodiment, the disease related to the activity of the NK cells is a disease showing abnormal activity of the NK cells, such as hypersensitive immune disorder, autoimmune disease, immunorejection, immunodeficiency disease, histiocytosis, cancer, type 2 diabetes, parasitosis, and viral disease. The hypersensitive immune disorder may include one or more selected from asthma and sinusitis, the autoimmune disease may include one or more selected from lupus, multiple sclerosis, type 1 diabetes, and rheumarthritis, or the histiocytosis may include one or more selected from HLH, XLP1, and XLP2. Hemophagocytic lymphohistiocytosis (HLH) may include Langerhans cell histiocytosis, erythrophagocytic lymphohistiocytosis (familial or sporadic), infection-associated hemophagocytic, virus-associated hemophagocytic, and histiocytosis with giant lymphadenopathy, or may include meaning of reticulohistiocytosis. The "cancer" may refer to a tumor, a blood cancer, or a solid cancer, and may include those that impair the synergistic activity of NK cells of an individual or those that do not cause the synergistic activity of NK cells as target cells under certain conditions. In an embodiment, the cancer may be selected from the group consisting of lung cancer, liver cancer, esophageal cancer, stomach cancer, large intestine cancer, small intestine cancer, pancreatic cancer, melanoma, breast cancer, oral cavity cancer, brain tumor, thyroid cancer, parathyroid cancer, renal cancer, cervical cancer, sarcoma, prostate cancer, urethral cancer, bladder cancer, testis cancer, blood cancer, lymphoma, skin cancer, psoriasis, and fibroadenoma. In an embodiment, the cancer may include pancreatic cancer or B cell lymphoma. In an embodiment, the viral disease may include hepatitis B. In an embodiment, the immunodeficiency may include DiGeorge syndrome or Chedial-Higashi syndrome.

Regarding the information on the NK cell activity-related disease, in a case where the activation of NK cells of an experimental group is not detected compared to normal NK cells, the NK cells are determined as abnormal NK cells, or in a case where NK cells that have lost activity for a specific receptor cause activity abnormally or do not cause any activity, the target cell is determined to be related to a specific disease.

Another aspect provides NK cells prepared by the method of proliferating NK cells.

Another aspect provides a cell therapy product including, as an active ingredient, the immune cell or a cell population thereof.

Another aspect provides a pharmaceutical composition for preventing or treating cancer or infectious diseases, the pharmaceutical composition including, as an active ingredient, the immune cell or a cell population thereof.

Another aspect provides use of the immune cell or a cell population thereof for manufacturing medicines.

Another aspect provides a method of treating a disease, the method including administering the immune cell or a cell population thereof to a subject.

The term "disease" as used in the present specification may refer to a pathological condition, particularly cancer, infectious disease, inflammatory disease, metabolic disease, autoimmune disorder, degenerative disease, apoptosis-related disease, and graft rejection.

The term "treatment" as used in the present specification refers to or includes alleviation, progression inhibition, or prevention of a disease, disorder, condition, or one or more symptoms thereof. The term "active ingredient" or "pharmaceutically effective amount" as used herein refers to the disease, disorder or condition , or any amount of a composition used in the practice of the invention provided herein sufficient to alleviate, inhibit a progression, or prevent a disease, disorder, condition, or one or more symptoms thereof.

The terms "administering", "introducing", and "grafting" as used in the present specification may be used interchangeably, and may be construed as arranging a composition according to an embodiment in a subject by a method or pathway that causes at least partial localization on a desired site. Cells or at least some of cell components of the composition according to an embodiment may be administered by any suitable pathway that delivers the cells or cell components to a desired location in a living subject. The survival period of the cells after administration to a subject may be as short as several hours, for example, from 24 hours to several days or as long as several years.

The term "isolated cell", for example, "isolated immune cell" or the like, as used in the present specification may refer to a cell substantially isolated from a tissue, for example, a hematoblast cell, from which the cell originates.

A way of administering the pharmaceutical composition according to an embodiment is not particularly limited, but depending on a desired way, the pharmaceutical composition may be administered orally or parenterally, such as intravenously, subcutaneously, intraperitoneally, inhalation, or topical application. A dosage may vary according to weight, age, gender, health status, and diet of a patient, an administration time, an administration method, an excretion rate, severity of a disease, and the like. A daily dosage refers to an amount of a therapeutic agent according to an aspect sufficient to treat an alleviated disease status by administration to a subject in need of treatment. An effective amount of a therapeutic agent may vary according to a particular compound, a disease status and severity thereof, and a subject in need of treatment, and may be generally determined by one of ordinary skill in the art. As a non-limiting example, the dosage of the composition according to an aspect for the human body may vary according to age, weight, gender, and a health status of a patient, a dosage form, and severity of a disease. Based on an adult patient weighing 70 kg, for example, about 1,000 cells/time to about 10,000 cells/time, about 1,000 cells/time to about 100,000 cells/time, about 1,000 cells/time to about 1000,000 cells/time, about 1,000 cells/time to about 10,000,000 cells/time, about 1,000 cells/time to about 100,000,000 cells/time, about 1,000 cells/time to about 1,000,000,000 cells/time, or about 1,000 cells/time to about 10,000,000,000 cells/time, may be fractionally administered once or several times a day at regular time intervals, or may be administered several times at regular time intervals.

The term "subject" may refer to a target in need of treatment for a disease, and more particularly, to a mammal including a human or a non-human primate, such as a mouse, a rat, a dog, a cat, a horse, a cow, and the like.

The pharmaceutical composition according to an embodiment may include a pharmaceutically acceptable carrier and/or additive. For example, sterile water, physiological saline, general buffer (e.g., phosphoric acid, citric acid, other organic acids, etc.), a stabilizer, a salt, an antioxidant (e.g., ascorbic acid, etc.), a surfactant, a suspending agent, an isotonic agent, or a preservative may be included. For topical administration, an organic material including a biopolymer, an inorganic material including hydroxyapatite, specifically a collagen matrix, a polylactic acid polymer or copolymer, a polyethylene glycol polymer or copolymer, and a chemical derivative thereof may be combined. When the pharmaceutical composition according to an embodiment is formulated in a dosage for suitable for injection, immune cells, immune cells, , or substances that increase the activity of the immune cells may be dissolved in the pharmaceutically acceptable carrier or frozen in a dissolved solution state.

The pharmaceutical composition according to an embodiment may include a suspending agent, a solubilizing agent, a stabilizer, an isotonic agent, a preservative, an adsorption inhibitor, a surfactant, a diluent, an excipient, a pH adjuster, an analgesic agent, a buffer, a reducing agent, an antioxidant, and the like may be appropriately included when necessary, according to the administration method or formulation of the pharmaceutical composition. Pharmaceutically acceptable carriers and agents suitable for the present invention, including those exemplified above, are described in detail in the document [Remington's Pharmaceutical Sciences, 19th ed., 1995]. The pharmaceutical composition may be formulated into a unit dosage form or prepared in a multi-dose container by formulating a pharmaceutically acceptable carrier and/or excipient according to the method easily carried out by a person having ordinary skill in the art to which the present invention pertains. Here, the formulation may be in the form of a solution, suspension, or emulsion in an oily or aqueous medium, or in the form of a powder, a granule, a tablet, or a capsule.

### ADVANTAGEOUS EFFECTS OF DISCLOSURE

Regarding genetically engineered cells according to an aspect, compared to non-genetically engineered cells, the proliferation and activity of NK cells can be increased by at least 2-fold to several-fold, so that the genetically engineered cell may be used as a cell therapeutic agent by proliferating NK cells.

### BRIEF DESCRIPTION OF DRAWINGS

FIG. 1A is a schematic diagram showing the gene of a lentivirus that produces mbIL-18 and mbIL-21 in order to prepare feeder cells according to an aspect.
FIG. 1B is a graph confirming the expression characteristics of mbIL-18 and mbIL-21 in feeder cells according to an aspect.
FIG. 2A is graphs showing the fold expansion (left) and purity (right) of NK cells when feeder cells according to an aspect and NK cells are cultured in a complete RPMI 1640 medium.
FIG. 2B is graphs showing the fold expansion (left) and purity (right) of NK cells when feeder cells according to an aspect and NK cells are cultured in a DS medium.
FIG. 2C is graphs comparing the fold expansion (left) and purity (right) of NK cells when feeder cells according to an aspect and NK cells are cultured in a complete RPMI 1640 medium and a DS medium.
FIGS. 3A and 3B are graphs comparing markers expressed in expanded NK cells, wherein the NK cells are expanded by using feeder cells (blue) according to an aspect and K562 feeder cells (red) in an RPMI 1640 medium and a DS medium.
FIG. 4A is a graph confirming the cytotoxicity of NK cells from healthy donors (HDs) expanded by using feeder cells according to an aspect.
FIG. 4B is a graph confirming the antibody-dependent cellular cytotoxicity (ADCC) of NK cells from HDs expanded by using feeder cells according to an aspect.
FIGS. 5A and 5B are graphs measuring the expression of CD107a in NK cells stimulated by feeder cells according to an aspect.
FIGS. 5C and 5D are graphs measuring the lysis rates of target cells by NK cells stimulated by feeder cells according to an aspect.

### MODE OF DISCLOSURE

Hereinafter, preferable Examples are presented to help understanding of the present disclosure. However, the following examples are only presented for easier understanding of the present disclosure, and the contents of the present disclosure are not limited by the following examples.

### [Examples]

### Example 1. Preparation of genetically engineered feeder cells

Feeder cells that express membrane bound interleukin-18 (mbIL-18) and membrane bound interleukin-21 (mbIL-21) were prepared.

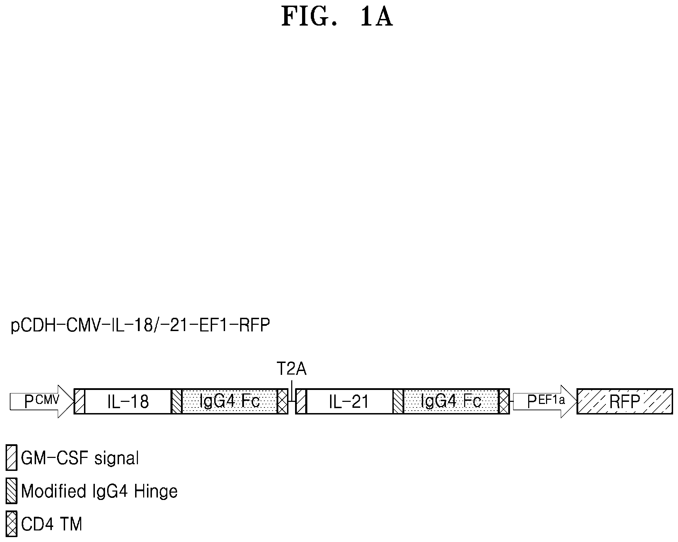

In detail, a human-derived mbIL18 gene (SEQ ID NO: 1) and a human-derived mblL21 gene (SEQ ID NO: 2) were cloned into a lentiviral vector, pCDH-CMV-RFP, to prepare a recombinant vector for producing a lentivirus (FIG. 1A). Then, the recombinant gene (pCDH-CMV-RFP-mbIL-1821) prepared for virus production was transfected into 293FT cells by using lipofectamin3000 (Invitrogen) together with a packaging vector. After a period of time, the medium was replaced with a fresh medium to culture the cells for 48 hours, and then the medium containing the virus was recovered. The recovered medium was centrifuged at a speed of 500 × g for 10 minutes, and only the pure medium containing the virus was separated through a 0.45 µm filter to produce an mbIL18-mbIL21 -expression lentivirus. Then, 1 ml of the mbIL18-mbIL21 expression lentivirus was dissolved in 9 ml of a medium containing K562 cells, and polybrene (8 µg/ml) was added thereto, followed by cell culture for 48 hours. Next, in order to select only infected cells, only cells expressing red fluorescence were selected by using an automated high-speed cytomer sorting system, and whether the mblL18-mblL21 was expressed was analyzed by using a fluorescence activated cell sorter (FACS), so as to produce a K562 cell line (hereinafter referred to as "K562-mbIL18-mbIL21").

### [Experimental Examples]

### Experimental Example 1. Cellular characteristics of genetically engineered feeder cells

In order to confirm the characteristics of the mbIL-18 and mbIL-21 expression of the genetically engineered feeder cells according to an aspect, the mRNA and surface protein expression levels were measured.

In detail, the total RNA of the K562-mbIL18-mbIL21 cell line and K562 feeder cell line prepared in Example 1 was isolated by using an RNeasy Mini Kit (Qiagen, Venlo, Netherlands) and then quantified by using an IMPLEN Nanophotometer P330 (IMPLEN, Munich, Germany). Afterwards, the isolated RNA was converted to cDNA by using a QuantiTect Reverse Transcription Kit (Qiagen), and PCR was performed thereon with a standard reaction volume of 20 µl by using a QuantiTect SYBR Green PCR Kit (Qiagen) and Rotor-Gene Q (Qiagen). Here, primers shown in Table 1 were used, and all experiments were repeated three times.

**Table 1**

| Gene | Direction | Primer sequence (5' to 3') | Accession No. |
|---|---|---|---|
| *IL-18* | Forward | CATTGACCAAGGAAATCGGC | NM_001562 |
| | Reverse | CACAGAGATAGTTACAGCCA TACC | |
| *IL-21* | Forward | AAGCTGAAGAGGAAACCACC | NM_021803 |
| | Reverse | TCTTTCTAGGAATTCTTTGGG TGG | |
| *GAPDH* | Forward | ACATCGCTCAGACACCATG | NM_002046 |
| | Reverse | TGTAGTTGAGGTCAATGAAG GG | |

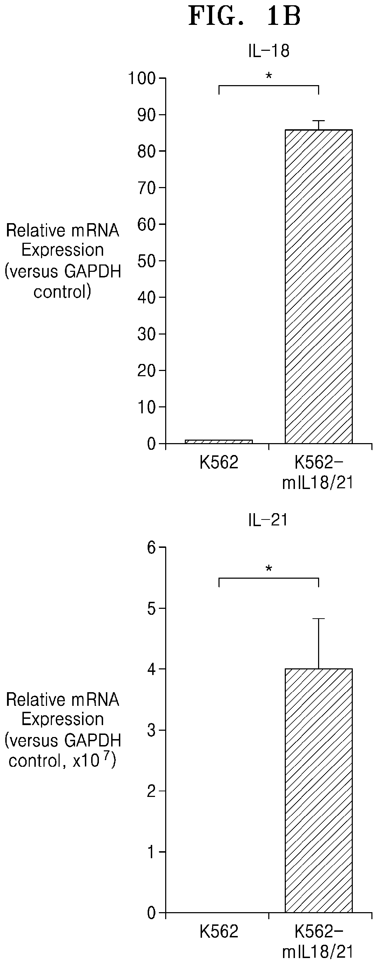

FIG. 1B is a graph confirming the expression characteristics of mbIL-18 and mbIL-21 in the feeder cells according to an aspect.

As a result, as shown in FIG. 1B, the mbIL-18 and mbIL-21 mRNA was detected in the feeder cells of Example 1, whereas the mbIL-18 and mbIL-21 mRNA was not detected in the K562 feeder cells. That is, it was confirmed that the genetically engineered feeder cells according to an embodiment had the expression characteristics of mbIL-18 and mblL-21.

### Experimental Example 2. Activation and expansion of NK cells by K562-mbIL18-mbIL21 cell

The activation and expansion of NK cells depending on a medium in which the genetically engineered feeder cells according to an aspect were included were confirmed.

### 2-1. Complete RPMI 1640 medium

In detail, the K562-mbIL18-mbIL21 cell line prepared in Example 1 was cultured in a T-75 flask containing 25 ml of complete RPMI 1640 medium at 37 °C in an incubator supplied with 5 % CO₂. Next, centrifugation was performed under 400 × g conditions for 3 minutes, and the cell pellets were resuspended in 5 ml of the complete RPMI 1640 medium to collect feeder cells. Then, in order to prevent excessive growth of the feeder cells, the feeder cells were irradiated with gamma rays at 100 gray (Gy) by using a Gammacell 3000 Elan radiator, and the irradiated feeder cells were used in subsequent experiments.

For isolation of peripheral blood mononuclear cells (PBMCs), whole blood of a healthy donor was diluted with PBS at a ratio of 1:2 (10 ml of whole blood:20 ml of PBS), and then overlaid on 15 ml Lymphoprep. Next, the centrifugation was performed without brake at a speed condition of 1200 × g for 25 minutes at room temperature (acceleration level: 1 and deceleration level: 0), and the cells were collected from a buffy coat layer and washed with PBS three times each at a speed of 400 × g for 7 minutes. 3×10⁶ of the isolated PBMCs and 0.5×10⁶ of the 100 Gy-irradiated K562-mbIL18-mbIL21 were inoculated onto a 24-well plate containing 1 ml of NK cell medium in each well. Then, 1 ml of NK cell medium containing 20 U/ml of IL-2 was added to each well so that the total medium volume of 2 ml/well and the final concentration of IL-2 was 10 U/ml. After mixing by pipetting gently, the cells were cultured in a 5 % CO₂ incubator at 37 °C. The culture was continued for 14 days. Meanwhile, 100 U/ml of IL-2 and 5 ng/ml of IL-15 were added to the medium on the 7th day of the culture, and the medium was replaced with a fresh medium every 2 to 3 days. Here, the K562 cells were used as a control group.

The expansion of the NK cells was confirmed by using fluorescein isothiocyanate (FITC)-conjugated mouse anti-human CD3 and PE-Cy5-conjugated mouse anti-human CD56 monoclonal antibodies. The purity of the NK cells that were treated with the K562 cells and the IL-2/IL-15 was considered as a baseline, and accordingly, the fold expansion for each treatment was confirmed.

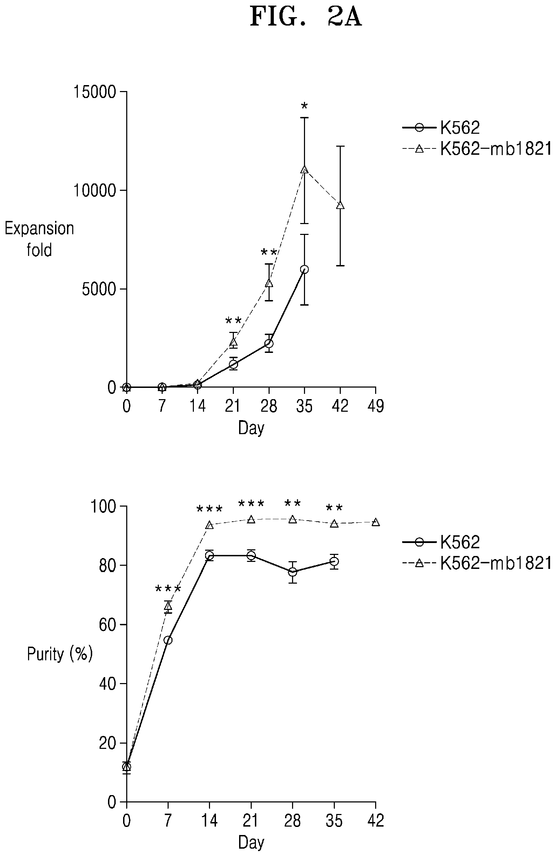

FIG. 2A is a graph showing the fold expansion (left) and purity (right) of the NK cells when the feeder cells according to an aspect and the NK cells were cultured in the complete RPMI 1640 medium.

As a result, as shown in FIG. 2A, it was confirmed that the expansion of the NK cells cultured with the K562 feeder cells increased until the 35th day after the culture, but did not occur thereafter. Meanwhile, it was confirmed that the NK cells cultured with the feeder cells of Example 1 showed an increasing pattern in the fold expansion until the 42nd day after the culture. In addition, it was confirmed that, as compared with the NK cells cultured with the K562 feeder cells, the NK cells cultured with the feeder cells of Example 1 had significantly high purity. That is, the feeder cells according to an aspect not only significantly increased the activity of NK cells, but also allowed long-term culture of NK cells, so that the NK cells may be mass-proliferated and utilized as cell therapeutic agents.

### 2-2. DMEM+Supplements (DS) medium

An experiment was performed in the same manner as in Experimental Example 2-1, except that a DS medium published by Miller group (JOURNAL OF HEMATOTHERAPY 4:149-158 (1995)) in which a DMEM culture medium was supplemented with 4.5 g/L of glucose and 584 mg/L of glutamine was used.

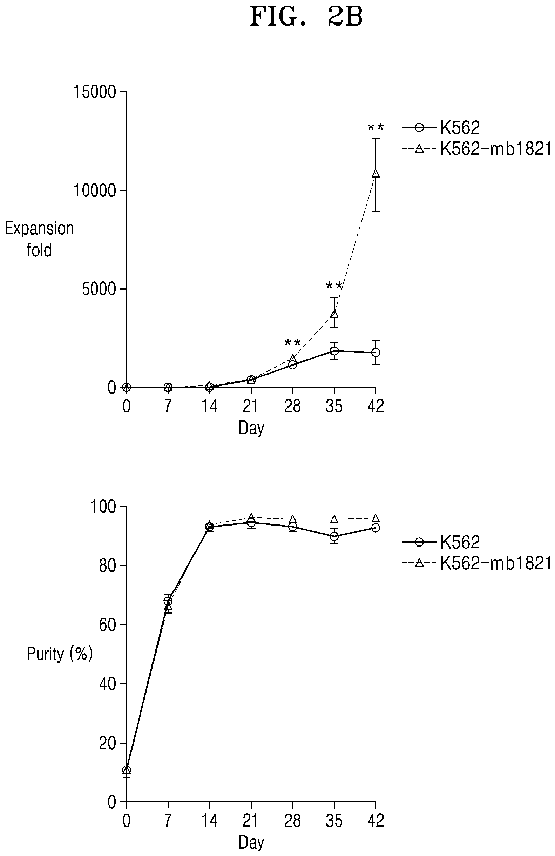

FIG. 2B is a graph showing the fold expansion (left) and purity (right) of NK cells when the feeder cells according to an aspect and NK cells were cultured in the DS medium.

As a result, as shown in FIG. 2B, it was confirmed that both the feeder cells of Example 1 and the cells of Comparative Example 1 increased the expansion fold of the NK cells until the 42nd day. However, it was confirmed that, as compared to the cells of Comparative Example 1, the fold expansion of the NK cells cultured with the feeder cells of Example 1 was significantly increased by about 2 times to about 6 times or more. In addition, it was confirmed that, as compared with the NK cells cultured with the K562 feeder cells, the NK cells cultured with the feeder cells of Example 1 had significantly high purity.

Based on the results above, the expansion efficiency of the NK cells in the RPMI 1640 medium was compared with that of the NK cells in the DS medium.

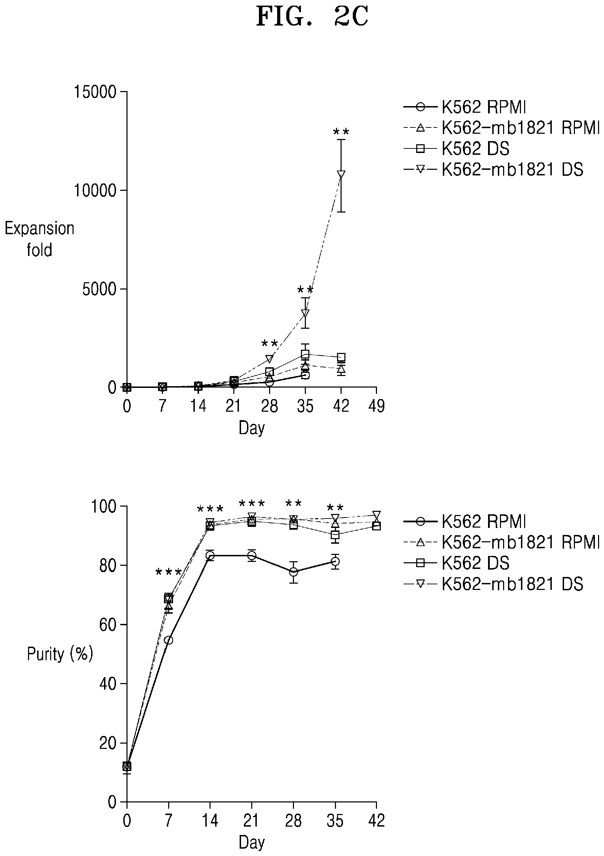

FIG. 2C is a graph comparing the fold expansion (left) and purity (right) of the NK cells when the feeder cells according to an aspect and the NK cells were cultured in the complete RPMI 1640 medium and the DS medium.

As a result, as shown in FIG. 2c, it was confirmed that the cells of Example 1 increased the fold expansion and purity of the NK cells as compared to the K562 feeder cells, regardless of the type of medium. However, when comparing the same cells cultured in different media, it was confirmed that the cells of Example 1 had significantly high fold expansion of the NK cells in the DS medium as compared to the NK cells in the complete RPMI 1640 medium. That is, the feeder cells according to an aspect were able to selectively expand NK cells according to the type of medium, and thus may have medium-selective characteristics.

### Experimental Example 3. Phenotypic characteristics of NK cells expanded by K562-mbIL18-mbIL21 cells

The phenotypic characteristics of the NK cells expanded by the K562-mbIL18-mblL2 cells in the RPMI 1640 medium and the DS medium were confirmed.

In detail, for isolation of PBMCs, whole blood of a healthy donor was diluted with PBS at a ratio of 1:2 (10 ml of whole blood:20 ml of PBS), and then overlaid on 15 ml Lymphoprep. Next, the centrifugation was performed without brake at a speed condition of 1200×g for 25 minutes at room temperature (acceleration level: 1 and deceleration level: 0), and the cells were collected from a buffy coat layer and washed with PBS three times each at a speed of 400×g for 7 minutes. Then, 2×10⁵ of expanded NK cells derived from a healthy donor were washed with an FACS buffer (PBS with 1 % FBS), and then, treated with APC-Cyanine7-conjugated mouse anti-human CD3 and PE-Cyanine7-conjugated anti-human CD56 membrane antibodies for 15 minutes. Cells were then collected and further stained with different fluorescence-conjugated anti-human CD16, CD57, CD69, NK2G2A, NK2G2C, NKG2D, DNAM-1, NKp30, NKp46, KIR2DL1, KIR2DL2/3, and KIR3DL1 membrane antibodies, each for 30 minutes. Afterwards, the cells were washed with FACS buffer, and data were acquired by using an FACS Calibur instrument and analyzed by using a Kaluza software.

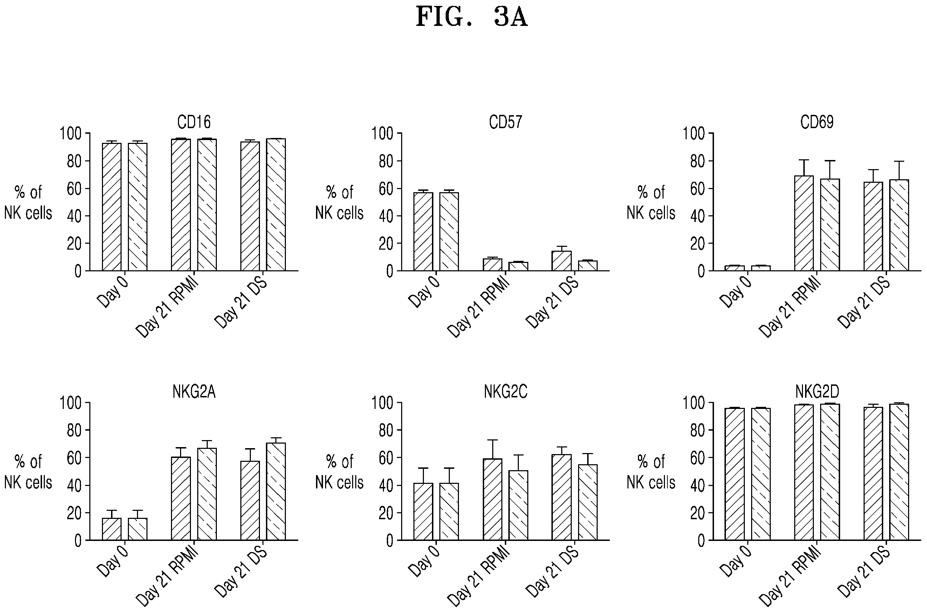

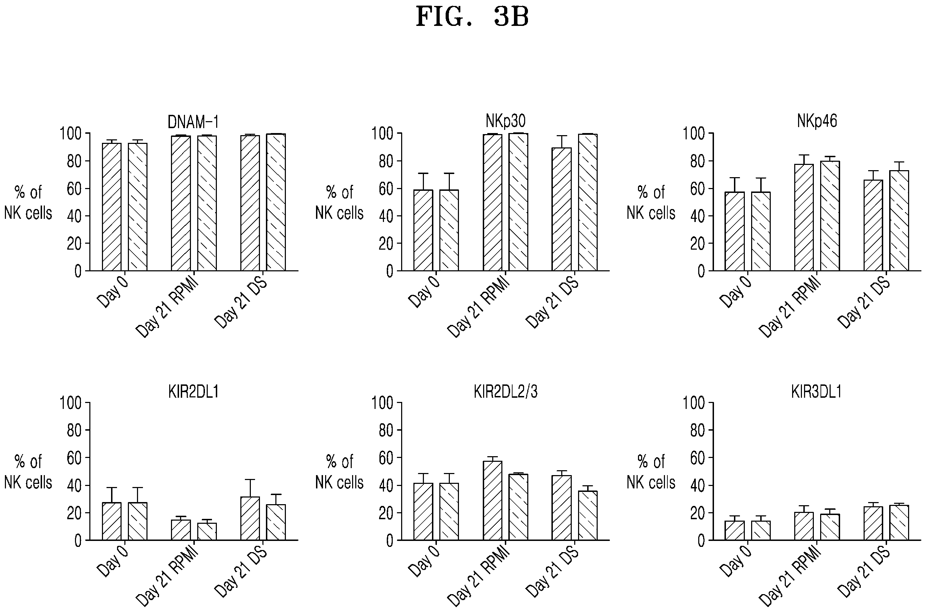

FIGS. 3A and 3B are graphs comparing markers expressed in the expanded NK cells, wherein the NK cells were expanded by using the feeder cells according to an aspect (blue) and the K562 feeder cells (red) in the RPMI 1640 medium and the DS medium.

As a result, as shown in FIGS. 3A and 3B, it was confirmed that the surface markers, such as CD16, CD57, CD69, NK2G2A, NK2G2C, NKG2D, DNAM-1, NKp30, NKp46, KIR2DL1, KIR2DL2/3, and KIR3DL1, were expressed in the NK cells expanded by using the feeder cells according to an aspect.

That is, it was confirmed that the NK cells expanded by the feeder cells according to an aspect had complete functional properties of the NK cells.

### Experimental Example 4. Confirmation of cytotoxicity of NK cells expanded by K562-mbIL18-mbIL21 cells

In order to confirm the efficacy of the NK cells, which were expanded by the feeder cells according to an aspect, as antibody therapeutic agents, the cytotoxicity of the NK cells against target cells on Day 14 was measured for 4 hours by a CFSE-based assay.

In detail, target cells were added to an FACS buffer, stained with 0.5 µM CFSE at 37 °C for 10 minutes, and washed twice with a complete medium (RPMI or DS). Then, 5×10⁴ of target cells were placed threefold on a 96-well round-bottom plate, and then mixed with the NK cells derived from healthy donors (HD) of Experimental Example 3 so that the effector-to-target (E:T) cells became 0.5:1, 1:1 and 2:1. The plate was centrifuged at a speed of 1,500 rpm for 3 minutes, and cultured for 4 hours at 37 °C in a 5 % CO₂ incubator. Next, the mixed cells were transferred to FACS tubes, and 1 µl of 1 mg/mL propidium iodide (PI) (Sigma Aldrich, St. Louis, MO, USA) was added to each tube. The cells were then collected in an FACS Calibur instrument and analyzed by using a Kaluza software. The percentage of dead target cells (CFSF-positive and PI-positive) was calculated after subtracting the percentage of spontaneously dead target cells.

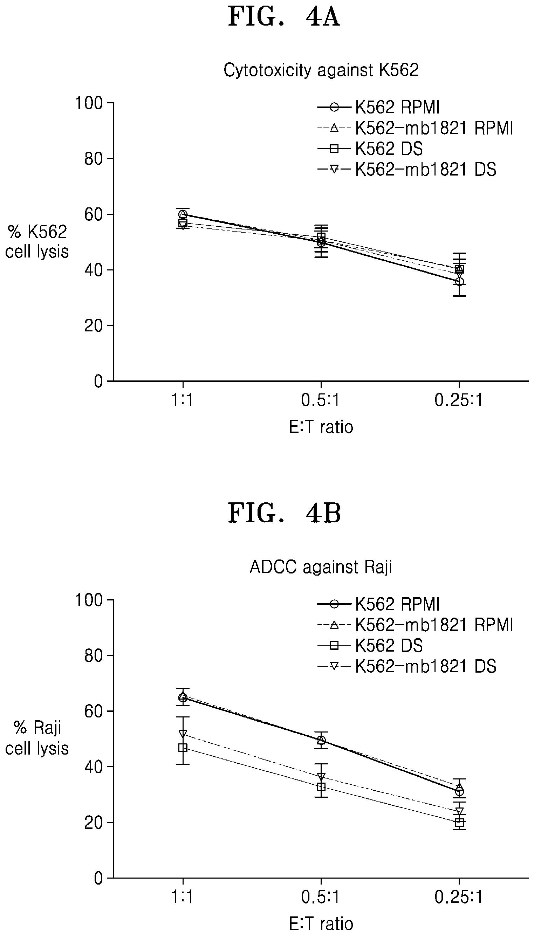

FIG. 4A is a graph confirming the cytotoxicity of the HD-derived NK cells that were expanded by using the feeder cells according to an aspect.

FIG. 4B is a graph confirming the antibody-dependent cellular cytotoxicity (ADCC) of the HD-derived NK cells that were expanded by using the feeder cells according to an aspect.

As a result, as shown in FIG. 4A, it was confirmed that the NK cells expanded by using the feeder cells of Example 1 showed the same cytotoxicity as the NK cells expanded by using the K562 feeder cells. It was also confirmed that the cytotoxicity of the NK cells cultured in the DS medium exhibited the same cytotoxicity as the NK cells cultured in the RPMI medium. In addition, as shown in FIG. 4B, it was confirmed that the NK cells expanded by using the feeder cells of Example 1 exhibited the similar ADCC activity against Rituximab-bound Raji cells as the NK cells expanded by using the K562 feeder cells. It was also confirmed that the ADCC activity of the NK cells cultured in the DS medium exhibited the similar ADCC activity as the NK cells cultured in the RPMI medium. That is, the NK cells expanded by the feeder cells according to an aspect exhibit the cytotoxicity, and thus can be utilized as antibody therapeutic agents.

### Experimental Example 5. Confirmation of activity of NK cells stimulated by K562-mbIL18-mbIL21 cells

### 5-1. Confirmation of degranulation activity by CD107a expression

In order to confirm the cell activity in whole blood of NK cells that were stimulated by the genetically engineered feeder cells according to an aspect, the expression level of CD107a on the surface of the NK cells proportional to the degranulation was measured. In detail, 100 µl of HD-derived whole blood of Experimental Example 3, 2×10⁵ of either of the K562 feeder cells or the K562-mbIL18-mblL21 feeder cell lines of Example 1, and PE-conjugated anti-human CD107a were cultured in an FACS tube. After 1 hour, Monensin and brefeldin A (BD Biosciences) were added and further cultured for 4 hours. Then, NK cells were obtained after staining with anti-human CD3 and CD56 antibodies, and the expression level of CD107a was measured.

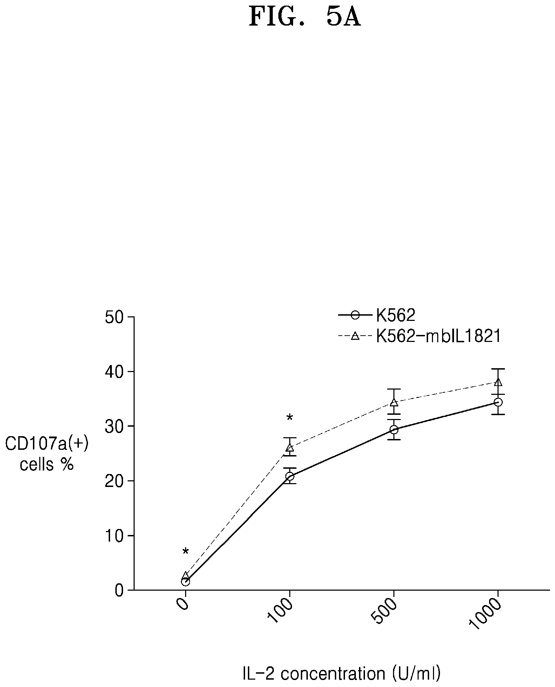

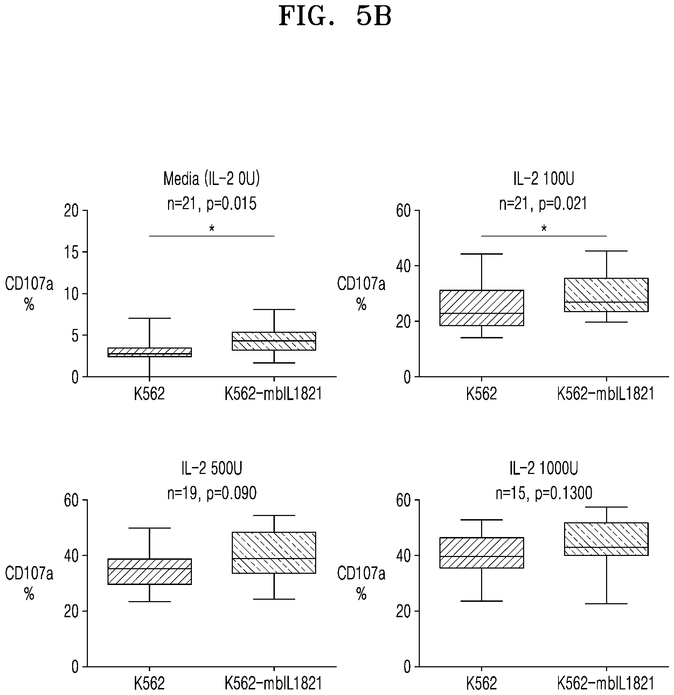

FIGS. 5A and 5B are graphs showing the measured expression level of CD107a in the NK cells that were stimulated by the feeder cells according to an aspect.

As a result, as shown in FIG. 5A, it was confirmed that the NK cells stimulated by the feeder cells of Example 1 increased the expression of CD107a in an IL-2 concentration-dependent manner. In detail, as shown in FIG. 5B, it was confirmed that the NK cells stimulated by the feeder cells of Example 1 showed significantly high expression levels of CD107a at the same concentration of IL-2 as compared to the NK cells stimulated by the K562 feeder cells.

That is, using the feeder cells according to an aspect to activate the NK cells can be more usefully utilized as a method of diagnosing the NK cell activity than using the K562 feeder cells.

### 5-2. Confirmation of cytotoxicity by cell lysis

In order to confirm the cellular activity in whole blood of NK cells that were stimulated by the genetically engineered feeder cells according to an aspect, the cytotoxicity of target cells was measured for about 16 hours to about 20 hours according to the CFSE-based assay. In detail, target cells (e.g., the K562 feeder cells or the feeder cells of Example 1) were added to a FACS buffer, stained with 0.5 µM CFSE at 37 °C for 10 minutes, and then washed twice with a complete medium (e.g., an RPMI medium or a DS medium). Then, 5×10⁴ of the target cells were placed threefold in FACS tubes, and the complete medium supplemented with IL-2 and the HD-derived whole blood were mixed therein to have a volume ratio of 200 ul, 100 ul, and 50 ul. The cells in the FACS tubes were cultured for about 16 hours to about 20 hours at 37 °C in a 5 % CO² incubator. Next, the mixed cells were transferred to FACS tubes, and 1 µl of 1 mg/mL PI (Sigma Aldrich, St. Louis, MO, USA) was added to each tube. The cells were then collected in an FACS Calibur instrument and analyzed by using Kaluza software. The percentage of dead target cells (CFSF-positive and PI-positive) was calculated after subtracting the percentage of spontaneously dead target cells.

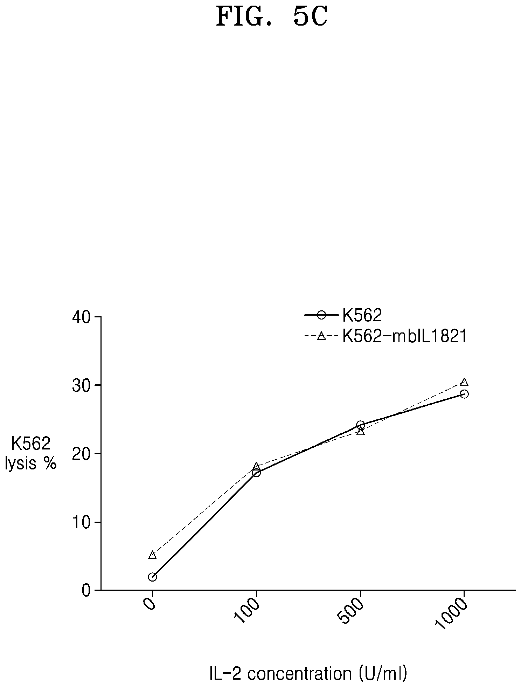

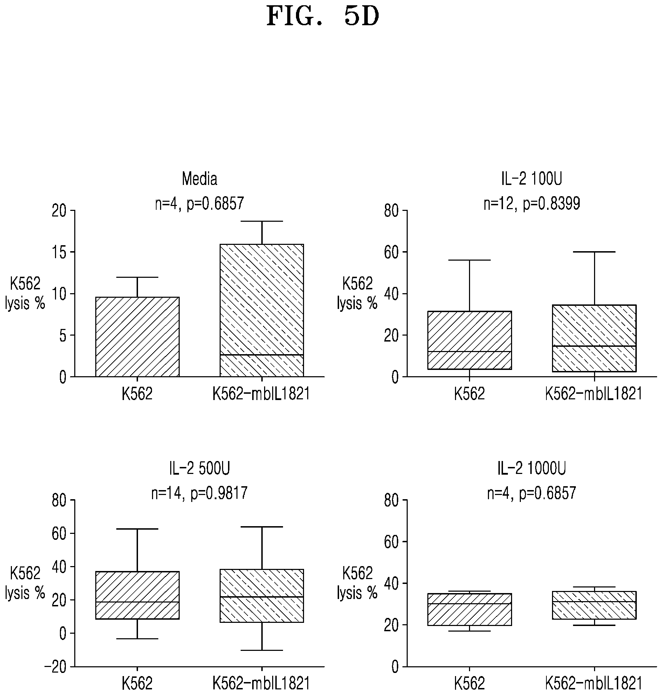

FIGS. 5C and 5D are graphs showing the measured lysis rates of the target cells by the NK cells stimulated by the feeder cells according to an aspect.

As a result, as shown in FIG. 5C, it was confirmed that the NK cells stimulated by the feeder cells of Example 1 increased the cell lysis rate of the target cells in an IL-2 concentration-dependent manner. In detail, as shown in FIG. 5D, it was confirmed that, as compared to the NK cells stimulated by the K562 feeder cells, the NK cells stimulated by the feeder cells of Example 1 showed significantly high cell lysis rate of the target cells at the same concentration of IL-2.

That is, using the feeder cells according to an aspect to activate the NK cells can be more usefully utilized as a method of diagnosing the NK cell activity than using the K562 feeder cells.

The foregoing descriptions are only for illustrating the present disclosure, and it will be apparent to a person having ordinary skill in the art to which the present invention pertains that the embodiments disclosed herein can be easily modified into other specific forms without changing the technical spirit or essential features. Therefore, it should be understood that Examples described herein are illustrative in all respects and are not limited.

## Claims

1. A feeder cell for culturing a natural killer (NK) cell, genetically engineered to express membrane bound interleukin-18 (mbIL-18) and membrane bound interleukin-21 (mbIL-21).

2. The feeder cell of claim 1, wherein the feeder cell is selected from the group consisting of K562, RPMI8866, EBV_LCL, 721.221, HFWT, and NK-92 cells.

3. The feeder cell of claim 1, wherein the feeder cell includes a nucleic acid encoding mbIL-18 and mblL-21.

4. A composition for culturing a natural killer (NK) cell, comprising the feeder cell of any one of claims 1 to 3.

5. A method of proliferating a natural killer (NK) cell, the method comprising:
obtaining a blood sample containing a population of NK cells; and
contacting at least a portion of the population of NK cells with a cell genetically engineered to activate NK cells, wherein the genetically engineered cell is genetically engineered to express membrane bound interleukin-18 (mblL-18) and membrane bound interleukin-21 (mbIL-21).

6. The method of claim 5, wherein the contacting comprises co-culturing the genetically engineered cell and the population of NK cells to expand a subpopulation of NK cells.

7. The method of claim 6, wherein the co-culturing is performed in the presence of cytokines.

8. The method of claim 7, wherein the cytokines comprise at least one selected from the group consisting of IL1, IL2, IL3, IL4, IL5, IL6, IL7, IL8 (CXCL8), IL9, IL10, IL11, IL12, IL13, IL14, IL15, IL16, IL17, IL18, IL19, IL20, IL21, IL22, IL23, IL24, IL25, IL26, IL27, IL28, IL29, IL30, IL31, IL32, IL33, IL35, and IL36.

9. The method of claim 8, wherein the cytokines comprise IL-18 and IL-21.

10. The method of claim 6, wherein the co-culturing is performed for 2 days to 30 days.

11. The method of claim 5, wherein the blood sample is a whole blood sample.

12. The method of claim 5, wherein the genetically engineered cell is treated with radiation in a range of about 50 gray (Gy) to about 300 Gy.

13. A method of examining activity of a natural killer (NK) cell, the method comprising:
obtaining a blood sample containing a population of NK cells;
contacting at least a portion of the population of NK cells with a cell genetically engineered to activate NK cells, to activate NK cells, the genetically engineered cell being genetically engineered to express membrane bound interleukin-18 (mblL-18) and membrane bound interleukin-21 (mbIL-21); and
analyzing an activation degree of the activated NK cells.

14. The method of claim 13, wherein the blood sample is a whole blood sample.

15. The method of claim 13, wherein the analyzing of the activation degree of the activated NK cells comprises measuring at least one selected from degranulation activity, cytotoxic activity, and cytokines secreted by NK cell stimulation.
